(19) Europäisches Patentamt
European Patent Office
Office européen des brevets

(11) Veröffentlichungsnummer: **0 017 623**
**B1**

(12) **EUROPÄISCHE PATENTSCHRIFT**

(45) Veröffentlichungstag der Patentschrift: **07.03.84**

(21) Anmeldenummer: **80810110.9**

(22) Anmeldetag: **02.04.80**

(51) Int. Cl.³: **C 08 G 59/50** // C07C93/04, C07C93/08, C07C93/12, C07C93/16, C07C93/187, C07C93/20, C07C121/34, C07C125/065, C07C125/073, C07C127/15, C07C127/19

(54) **Dimethylaminoderivate enthaltende härtbare Gemische.**

(30) Priorität: **10.04.79 CH 3405/79**

(43) Veröffentlichungstag der Anmeldung: **15.10.80 Patentblatt 80/21**

(45) Bekanntmachung des Hinweises auf die Patenterteilung: **07.03.84 Patentblatt 84/10**

(84) Benannte Vertragsstaaten: **AT CH DE FR GB IT LI NL SE**

(56) Entgegenhaltungen:
**CH - A - 362 526**
**DE - A - 2 840 930**
**DE - C - 887 650**
**DE - C - 961 029**
**DE - C - 1 034 616**
**DE - C - 1 053 492**
**US - A - 2 965 672**
**US - A - 3 081 310**

(73) Patentinhaber: **CIBA-GEIGY AG, Postfach, CH-4002 Basel (CH)**

(72) Erfinder: **Zondler, Helmut, Dr., Oberwilerstrasse 49, CH-4103 Bottmingen (CH)**
Erfinder: **Moser, Roland, Säckingerstrasse 10, CH-4058 Basel (CH)**

**EP 0 017 623 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Dimethylaminoderivate enthaltende härtbare Gemische

Die vorliegende Erfindung betrifft härtbare Gemische mit (Dimethylaminoalkyl)-carbonsäureestern, -carbaminsäureestern, -äthern und -harnstoffen als aminischen Härtern für Polyepoxidverbindungen mit durchschnittlich mehr als einer Epoxidgruppe im Molekül.

Epoxidharze finden eine breite Verwendung in verschiedenen technischen Bereichen wie z.B. zum Oberflächenschutz, in der Elektro- und Bauindustrie, als Klebemittel, Verbundwerkstoffe und Werkzeugharze. Neben verschiedenen üblichen Zusätzen enthalten die Epoxidharze Härter, deren Zusammensetzung z.B. auf die Verarbeitungsbedingungen, den Anwendungsbereich und die Produkteigenschaft abgestimmt werden müssen.

Es ist bekannt, N,N-Dialkyl-1,3-propylendiamine als Härter einzusetzen, und zwar für sich alleine (DE-PS 961 029) oder zusammen mit «Polyamidharzen» (CH-PS 362 526). Obwohl sich diese 1,3-Propylendiamine als gute Härter erwiesen haben, ist ihre Verwendung nicht ohne Nachteile. Zum einen handelt es sich um relativ niedrig siedende Substanzen, die bei der Verarbeitung zur Herstellung von Epoxidharzen teilweise verdampfen und zu nicht unbedenklichen Geruchsbelästigungen führen. Diese können durch die Verwendung von Absaugvorrichtungen nur unvollständig behoben werden. Anderseits wird oft durch den Feuchtigkeits- und Kohlendioxidgehalt der Luft eine Carbonatabscheidung an Verarbeitungsmaschinen beobachtet.

N,N-Dialkyl-1,3-propylendiamine sind noch relativ reaktiv, so dass nach dem Vermischen der Komponenten frühzeitig der Viskositätsbereich erreicht wird, bei dem die Mischung nicht mehr verarbeitbar ist. Diese Viskosität wird oft durch die Gelierzeit angegeben, die in diesem Fall also noch als zu gering angesehen wird, was anwendungstechnische Nachteile bedeutet.

Es ist auch schon vorgeschlagen worden, N,N-Dimethyl-1,3-propylendiamin als Härter in solchen Polyepoxidzusammensetzungen zu verwenden, die als Klebemittel eingesetzt werden. Obwohl hiermit sehr gute Haftfestigkeiten erzielt werden, besteht das Bedürfnis, die Klebewirkung zu verbessern, um den Anwendungsbereich erweitern zu können.

Aufgabe vorliegender Erfindung ist es, härtbare Gemische aus Polyepoxidverbindungen mit einem Gehalt an aminischem Härter bereitzustellen, die längere Härtungszeiten und auch eine ähnliche oder verbesserte Klebewirkung beim Einsatz als Klebemittel aufweisen. Bevorzugt sollen die aminischen Härter schwerflüchtiger sein als z.B. N,N-Dimethyl-1,3-propylendiamin.

Gegenstand vorliegender Erfindung ist ein härtbares Gemisch, enthaltend (a) eine Polyepoxidverbindung mit durchschnittlich mehr als einer Epoxidverbindung im Molekül und (b) einen Härter, dadurch gekennzeichnet, dass der Härter ein Dimethylaminoderivat der Formel I

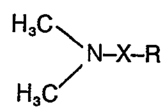 (I)

worin

X 1,3-Propylen oder Äthylen oder durch Alkyl substituiertes Äthylen ist und

R den Formeln

$$(CH_3)_2N{-}X{-}O\overset{\overset{O}{\|}}{C}O\ (II),\ R^1\overset{\overset{O}{\|}}{C}O\ (III),\ (CH_3)_2N{-}X{-}O\overset{\overset{O}{\|}}{C}Y\overset{\overset{O}{\|}}{C}O\ (IV),$$

$$R^2R^3N\overset{\overset{O}{\|}}{C}O\ (V),\ (CH_3)_2N{-}X{-}O\overset{\overset{O}{\|}}{C}NH{-}Y{-}HN\overset{\overset{O}{\|}}{C}O\ (VI)\ OR^4\ (VII)$$

oder $OR^{11}OXN(CH_3)_2$ (XI) entspricht,

worin $R^1$ Alkyl, Cycloalkyl, unsubstituiertes oder mit Halogen oder $C_1$–$C_4$-Alkyl substituiertes Aryl oder Aralkyl bedeutet, Y eine direkte Bindung, Alkylen, Alkenylen, Cycloalkylen oder Arylen ist, $R^2$ ein Wasserstoffatom ist oder die Bedeutung von $R^3$ hat und $R^3$ Alkyl, Cycloalkyl oder mit $C_1$–$C_4$-Alkyl oder Halogen substituiertes oder unsubstituiertes Aryl oder Aralkyl darstellt und $R^4$ Alkyl, Alkenyl, Cycloalkyl, Cycloalkylalkylen, unsubstituiertes oder mit $C_1$–$C_4$-Alkyl oder Halogen substituiertes Aryl oder Aralkyl, $CH_2\underset{R^5}{\underset{|}{C}}H{-}Z$ oder $\underset{R^5}{\underset{|}{C}}HCH_2{-}Z$ ist, worin $R^5$ ein Wasserstoffatom oder Methyl und Z CN, $CH_2NH_2$ oder $CH_2N(CH_3)_2$ bedeutet, wobei X zusätzlich Neopentylen ist, wenn R den Rest $OR^4$ darstellt, $R^{11}$ Alkylen, Cycloalkylen oder Arylen ist, oder

X Äthylen, 1,3-Propylen oder in 1- oder 2-Stellung durch Methyl substituiertes 1,3-Propylen ist und

R den Formeln

$$NR^6\overset{\overset{O}{\|}}{C}OR^7\ \ (VIII),\quad R^8R^9N\overset{\overset{O}{\|}}{C}NR^{10}\ \ (IX)\quad \text{oder}\quad (CH_3)_2N{-}X{-}NH\overset{\overset{O}{\|}}{C}NH{-}(Y{-}NH\overset{\overset{O}{\|}}{C}NH)_n\ \ (X)\ \ \text{entspricht,}$$

worin X und Y unabhängig die zuvor angegebene Bedeutung haben, n 0 oder 1 ist, $R^6$ ein Wasserstoffatom, Alkyl, Cycloalkyl, β-Cyanoäthyl oder in 1- oder 2-Stellung durch Methyl substituiertes β-Cyanoäthyl ist, $R^7$ Alkyl, Alkenyl, Cycloalkyl, Aryl, Aralkyl, Alkaryl oder Alkaralkyl bedeutet, $R^8$ und $R^{10}$ ein Wasserstoffatom und $R^9$ Alkyl, Cycloalkyl, Aryl, Aralkyl, Alkaryl, Alkaralkyl, 3-(Dimethylamino)-propyl oder $R^8$ und $R^9$ unabhängig voneinander $C_1$–$C_4$-Alkyl oder Phenyl und $R^{10}$ ein Wasserstoffatom oder $R^8$ ein Wasserstoffatom, $R^9$ Alkyl, Cycloalkyl, Aryl, Aralkyl, Alkaryl oder Aralkaryl und $R^{10}$ β-Cyanoäthyl oder in 1- oder 2-Stellung durch Methyl substituiertes β-Cyanoäthyl sind, ist.

Eine bevorzugte Gruppe sind jene Derivate, in denen in Formel I X gegebenenfalls durch $C_1$–$C_{12}$-Alkyl, vorzugsweise $C_1$–$C_4$-Alkyl substituiertes Äthylen bedeutet und R den Formeln II–VII entspricht.

Eine andere bevorzugte Gruppe sind jene Derivate, in denen in Formel I X Äthylen oder 1,3-Propylen ist und R den Formeln VIII–X entspricht.

$R^1$ in Formel III ist vorzugsweise $C_1$–$C_{12}$-Alkyl, besonders $C_1$–$C_6$-Alkyl, $C_5$–$C_7$-Cycloalkyl, oder unsubstituiertes oder mit $C_1$–$C_4$-Alkyl oder Halogen, besonders Chlor, substituiertes Phenyl oder Benzyl.

In Formel V sind $R^2$ bevorzugt ein Wasserstoffatom und $R^3$ $C_1$–$C_{12}$-Alkyl, besonders $C_1$–$C_4$-Alkyl, $C_5$–$C_7$-Cycloalkyl, unsubstituiertes oder mit $C_1$–$C_4$-Alkyl oder Halogen, besonders Chlor substituiertes Phenyl oder Benzyl oder $R^2$ und $R^3$ unabhängig voneinander $C_1$–$C_4$-Alkyl, $C_5$–$C_7$-Cycloalkyl oder Phenyl.

In Formel VII ist $R^4$ vorzugsweise $C_1$–$C_{18}$-, besonders $C_1$–$C_{12}$-Alkyl oder -Alkenyl, $C_5$–$C_8$-Cycloalkyl, $C_6$–$C_{16}$-Aryl, $C_7$–$C_{16}$-Aralkyl, β-Cyanoäthyl, γ-Aminopropyl oder γ-Dimethylaminopropyl.

In Formel IV ist Y bevorzugt eine direkte Bindung und in den Formeln IV und X bevorzugt $C_1$–$C_{12}$-, besonders $C_1$–$C_6$-Alkylen oder Alkenylen, $C_5$–$C_7$-Cycloalkylen oder $C_6$–$C_{12}$-Arylen.

$R^6$ in Formel VIII enthält in seiner Bedeutung als Alkyl bevorzugt 1–4 C-Atome und in seiner Bedeutung als Cycloalkyl bevorzugt 6 C-Atome.

In Formel VIII ist $R^7$ bevorzugt $C_1$–$C_{18}$-, besonders $C_1$–$C_{12}$- und insbesondere $C_1$–$C_6$-Alkyl oder -Alkenyl, $C_5$–$C_7$-Cycloalkyl, $C_6$–$C_{12}$-Aryl, $C_7$–$C_{16}$-Aralkyl oder -Alkaryl oder $C_8$–$C_{16}$-Alkaralkyl.

In Formel IX ist eine bevorzugte Gruppe jene, in der $R^8$ und $R^{10}$ ein Wasserstoffatom und $R^9$ $C_1$–$C_{12}$-, besonders $C_1$–$C_6$-Alkyl, $C_5$–$C_7$-Cycloalkyl, $C_6$–$C_{12}$-Aryl, $C_7$–$C_{16}$-Aralkyl oder Alkaryl oder $C_8$–$C_{16}$-Aralkaryl sind.

Eine andere bevorzugte Gruppe der Formel IX ist jene, in der $R^8$ und $R^9$ unabhängig voneinander $C_1$–$C_4$-Alkyl oder Phenyl und $R^{10}$ ein Wasserstoffatom sind.

Eine weitere bevorzugte Gruppe der Formel IX ist jene, in der $R^8$ ein Wasserstoffatom, $R^9$ $C_1$–$C_{12}$-, besonders $C_1$–$C_6$-Alkyl, $C_5$–$C_7$-Cycloalkyl, $C_6$–$C_{12}$-Aryl, $C_7$–$C_{16}$-Alkaryl oder -Aralkyl oder $C_8$–$C_{16}$-Alkaralkyl und $R^{10}$ β-Cyanoäthyl oder in 1- oder 2-Stellung durch Methyl substituiertes β-Cyanoäthyl sind.

In XI ist $R^{11}$ bevorzugt $C_1$–$C_{12}$-Alkylen, $C_5$–$C_7$-Cycloalkylen oder $C_6$–$C_{12}$-Arylen.

Beispiele für Alkyl und Alkenyl, die linear oder verzweigt sein können, sind:
Methyl, Äthyl, Propyl, i-Propyl, n-, i- oder t-Butyl, Pentyl, Hexyl, Heptyl, Octyl, Nonyl, Decyl, Undecyl,
Dodecyl, Tetradecyl, Octadecyl, Äthenyl, Propenyl,
i-Propenyl, 1-Butenyl, 2-Butenyl, Pentenyl, Hexenyl, Octenyl,
Dodecenyl, Octadecenyl.

Beispiele für Alkylen und Alkenylen, die linear oder verzweigt sein können, sind:
Methylen, Äthylen, 1,2- oder 1,3-Propylen, 1,2-, 1,3- oder
1,4-Butylen, Pentylen, Hexylen, 2-Äthylbutylen,
Octylen, Dodecylen, Octyläthylen, Dodecyläthylen,
Äthenylen, Methyläthenylen, Dimethyläthenylen,
Propenylen, Butenylen, Hexenylen, Äthyläthenylen,
Octyläthenylen. Cycloalkylen kann bevorzugt Cyclopentylen, Cycloheptylen, Cyclooctylen und besonders Cyclohexylen sein. Arylen ist bevorzugt o-, m- und p-Phenylen und Naphthylen, das mit $C_1$–$C_4$-Alkyl substituiert sein kann.

Cycloalkyl, das durch Alkylgruppen mit 1–4 C-Atomen substituiert sein kann, ist z.B. Cyclopentyl, Cycloheptyl, Cyclooctyl und besonders Cyclohexyl.

Aryl ist für sich oder in den Aryl enthaltenden Gruppen bevorzugt Naphthyl und besonders Phenyl. Beispiele für Aralkyl, Alkaryl oder Alkaralkyl sind
Benzyl, Methylphenyl, Methylnaphthyl, Dimethylphenyl,
Diäthylphenyl, Äthylphenyl, Butylphenyl,
Dibutylphenyl, Octylphenyl, Nonylphenyl,
Äthylmethylphenyl, Benzyl, β-Phenyläthyl,
γ-Phenylpropyl, Methylbenzyl, Dimethylbenzyl,
Äthylbenzyl, Butylbenzyl, Dibutylbenzyl oder Nonylbenzyl.

Die Herstellung der Dimethylaminoderivate der Formel I erfolgt nach bekannten Methoden in hierfür üblichen Einrichtungen.

Die Äther werden z.B. erhalten, indem man Alkohole der Formel $(CH_3)_2N$–X–OH mit Alkoholen der Formel R–OH in Gegenwart von wasserentziehenden Mitteln veräthert oder mit Halogeniden der Formel R–A, worin A Halogen, bevorzugt Chlor oder Brom ist, gegebenenfalls in Gegenwart von Katalysatoren umsetzt oder indem man einen Alkohol der Formel $(CH_3)_2N$–X–OH an entsprechende olefinisch ungesättigte Verbindungen anlagert.

Die Ester erhält man durch Veresterung oder Umsetzung eines Alkoholes der Formel $(CH_3)_2N$–X–OH mit Mono- oder Dicarbonsäuren oder deren esterbildenden Derivaten, z.B. Anhydriden, Estern oder Säurehalogeniden, gegebenenfalls in Gegenwart von Katalysatoren.

Die Carbaminsäureester erhält man durch die Anlagerung von Alkoholen der Formel $(CH_3)_2N-X-OH$ an Mono- oder Diisocyanate oder durch die Umsetzung von ca. 1 Mol Dicarbonsäureester mit ca. 1 Mol Amin der Formel $(CH_3)_2N-X-\underset{R^6}{\underset{|}{N}}H$. N-Substituenten $R^2$, $R^3$ oder $R^6$ können auch nach diesen Reaktionen durch Umsetzung mit entsprechenden Halogeniden oder olefinisch ungesättigten Verbindungen eingeführt werden.

Die Harnstoffe können durch Umsetzung von Mono- oder Diisocyanaten mit Aminen der Formel $(CH_3)_2N-X-\underset{R^{10}}{\underset{|}{N}}H$ erhalten werden oder durch die Umsetzung dieser Amine mit gegebenenfalls mit $R^8$- oder $R^9$-substituierten Harnstoffen. Die Gruppen $R^8$, $R^9$ und $R^{10}$ können auch nach dieser Reaktion durch Umsetzung mit entsprechenden Halogeniden oder olefinisch ungesättigten Verbindungen eingeführt werden.

Die als Ausgangsverbindungen verwendeten Alkohole und Amine sind käuflich oder leicht durch Anlagerung von Epoxiden bzw. Acrylnitril, Methacrylnitril oder Crotonitril an Dimethylamin erhältlich, wobei die erhaltenen Nitrile anschliessend zu Aminen hydriert werden.

Die Dimethylaminoderivate der Formel I werden den härtbaren Gemischen im allgemeinen in Mengen von 0,1 bis 30 Gewichtsteilen, bevorzugt 0,5 bis 20 und insbesondere 1 bis 15 Gewichtsteilen zugegeben, bezogen auf die vorhandene Polyepoxidverbindung. Sie können für sich alleine oder zusammen mit anderen Härtern eingesetzt werden, wobei sie dann oft als Härtungsbeschleuniger wirken. Bekannte Härter sind z.B. Säuren wie Di- und Polycarbonsäuren, Carbonsäureanhydride, mehrwertige Alkohole und Phenole, Polyamide, Melamin-Formaldehyd- sowie Harnstoff-Formaldehyd-Kondensate, Polyamine, Polyisocyanate, Phenoplast- und Aminoplastvorkondensate. Andere geeignete Härtungsmittel sind z.B. Polyaminoamide, die aus di- oder trimerisierten Fettsäuren und aliphatischen Polyaminen hergestellt werden (vgl. CH-PS 362 526). Bei der Verwendung als Härtungsbeschleuniger bzw. zusammen mit anderen Härtern genügt im allgemeinen eine Menge von 0,1 bis 5 Gewichtsteilen, bezogen auf die Polyepoxidverbindung.

Die Dimethylamine können auch in Form von Addukten eingesetzt werden, z.B. mit flüssigem Butadien-Nitril-Copolymer, das endständige Carboxylgruppen aufweist. Sofern die Härter an N-Atome gebundene aktive Wasserstoffatome aufweisen, so kommen bevorzugt auf 0,5 bis 1,5 Äquivalente, insbesondere etwa 1 Äquivalent dieser H-Atome, 1 Äquivalent Epoxidgruppen in der härtbaren Mischung.

Als Polyepoxidverbindungen kommen für die erfindungsgemässen härtbaren Gemische vor allem solche mit durchschnittlich mehr als einer an ein Heteroatom (z.B. Schwefel, vorzugsweise Sauerstoff oder Stickstoff) gebundenen Glycidylgruppe, β-Methylglycidylgruppe oder 2,3-Epoxycyclopentylgruppe in Frage; genannt seien insbesondere Bis-(2,3-epoxycyclopentyl)äther; Di- bzw. Polyglycidyläther von mehrwertigen aliphatischen Alkoholen, wie 1,4-Butandiol, oder Polyalkylenglykolen, wie Polypropylenglykole; Di- oder Polyglycidyläther von cycloaliphatischen Polyolen, wie 2,2-Bis(4-hydroxycyclohexyl)-propan; Di- bzw. Polyglycidyläther von mehrwertigen Phenolen, wie Resorcin, Bis-(p-hydroxyphenyl)-methan, 2,2-Bis-(p-hydroxy-phenyl)-propyl (= Diomethan), 2,2-Bis-(4′-hydroxy-3′,5′-dibromphenyl)-propan, 1,1,2,2-Tetrakis-(p-hydroxylphenyl)-äthan, oder von unter sauren Bedingungen erhaltenen Kondensationsprodukten von Phenolen mit Formaldehyd, wie Phenol-Novolake und Kresol-Novolake; Di- bzw. Poly(β-methylglycidyl)-äther der oben angeführten mehrwertigen Alkohole oder mehrwertigen Phenole; Polyglycidylester von mehrwertigen Carbonsäuren, wie Phthalsäure, Terephthalsäure, $\Delta^4$-Tetrahydrophthalsäure und Hexahydrophthalsäure; N-Glycidylderivate von Aminen, Amiden und heterocyclischen Stickstoffbasen, wie
N,N-Diglycidylanilin, N,N-Diglycidyltoluidin,
N,N,N′,N′-Tetraglycidyl-bis-(p-aminophenyl)-methan; Triglycidyl-isocyanuarat; N,N′-Diglycidyläthylenharnstoff;
N,N′-Diglycidyl-5,5-dimethylhydantoin,
N,N′-Diglycidyl-5-isopropyl-hydantoin;
N,N′-Diglycidyl-5,5-dimethyl-6-isopropyl-5,6-dihydro-uracil.

Gewünschtenfalls kann man den Polyepoxiden zur Herabsetzung der Viskosität aktive Verdünner, wie z.B. Styroloxid, Butylglycidyläther, Isooctylglycidyläther, Phenylglycidyläther, Kresylglycidyläther, Glycidylester von synthetischen, hochverzweigten, in der Hauptsache tertiären aliphatischen Monocarbonsäuren («CARDURA E») zusetzen.

Die Härtung der erfindungsgemässen härtbaren Mischungen zu Formkörpern und dergleichen erfolgt zweckmässig im Temperaturintervall von 20 bis 160 °C. Man kann die Härtung in bekannter Weise auch zwei- oder mehrstufig durchführen, wobei die erste Härtungsstufe bei niedrigerer Temperatur und die Nachhärtung bei höherer Temperatur durchgeführt wird.

In manchen Fällen kann es gewünscht werden, die Gelier- bzw. -Härtungszeiten der erfindungsgemässen Gemische zu verkürzen. Hierzu können bekannte Beschleuniger für die Amin-Härtung, z.B. Mono- oder Polyphenole, wie Phenol oder Diomethan, Salicylsäure oder Salze der Rhodanwasserstoffsäure, wie $NH_4SCN$, zugesetzt werden.

Die erfindungsgemässen härtbaren Mischungen aus Polyepoxidverbindungen und einem Dimethylaminoderivat der Formel I können ferner vor der endgültigen Härtung in irgendeiner Phase mit üblichen Modifizierungsmitteln, wie Streck-, Füll- und Verstärkungsmitteln, Pigmenten, Farbstoffen, organischen Lösungsmitteln, Weichmachern, Verlaufmitteln, Thixotropiermitteln, flammhemmenden Stoffen, Formtrennmitteln versetzt werden.

Als Streckmittel, Verstärkungsmittel, Füllmittel und Pigmente, die in den erfindungsgemässen härtbaren Mischungen eingesetzt werden können, seien z.B. genannt: Steinkohlenteer, Bitumen, flüssige Cumaron-Inden-Harze, Textilfasern, Glasfasern, Asbestfasern, Borfasern, Kohlenstofffasern, Cellulose, Polyäthylenpulver, Polypropylenpulver; Quarzmehl; mineralische Silikate, wie Glimmer, Asbestmehl, Schiefermehl; Kaolin, Aluminiumoxidtrihydrat, Kreidemehl, Gips, Antimontrioxid, Bentone, Kieselsäureaerogel («AEROSIL»), Lithopone, Schwerspat, Titandioxid, Russ, Graphit, Oxidfarben, wie Eisenoxid, oder Metallpulver, wie Aluminiumpulver oder Eisenpulver.

Als organische Lösungsmittel eignen sich für die Modifizierung der härtbaren Mischungen z.B. Toluol, Xylol, n-Propanol, Butylacetat, Aceton, Methyläthylketon, Diacetonalalkohol, Äthylenglykolmonomethyläther, -mono-äthyläther und -monobutyläther.

Als Weichmacher können für die Modifizierung der härtbaren Mischungen z.B. Dibutyl-, Dioctyl- und Dinonylphthalat, Trikresylphosphat, Trixylenylphosphat, Diphenyloxyäthylformal und Polypropylenglykole eingesetzt werden.

Als Verlaufmittel («flow control agents») beim Einsatz der härtbaren Mischungen speziell im Oberflächenschutz, kann man z.B. Silicone, flüssige Acrylharze, Celluloseacetobutyrat, Polyvinylbutyral, Wachse, Stearate usw. (welche z.T. auch als Formtrennmittel Anwendung finden) zusetzen.

Speziell für die Anwendung auf dem Lackgebiet können ferner die Polyepoxidverbindungen in bekannter Weise mit Carbonsäuren, wie insbesondere höheren ungesättigten Fettsäuren, partiell verestert sein. Es ist ferner möglich, solchen Lackharzformulierungen andere härtbare Kunstharze, z.B. Phenoplaste oder Aminoplaste, zuzusetzen.

Die Herstellung der erfindungsgemässen härtbaren Mischungen kann in üblicher Weise mit Hilfe bekannter Mischaggregate (Rührer, Kneter, Walzen) erfolgen.

Die erfindungsgemässen härtbaren Epoxidharzmischungen finden ihren Einsatz vor allem auf den Gebieten des Oberflächenschutzes, der Elektrotechnik, der Laminierverfahren, der Klebstofftechnik und im Bauwesen. Sie können in jeweils dem speziellen Anwendungszweck angepasster Formulierung, im ungefüllten oder gefüllten Zustand, gegebenenfalls in Form von Lösungen oder Emulsionen, als Anstrichmittel, Lacke, Pressmassen, Spritzgussformulierungen, Tauchharze, Giessharze, Imprägnierharze, Bindemittel, als Werkzeugharze, Laminierharze, Dichtungs- und Spachtelmassen, Bodenbelagsmassen und Bindemittel für mineralische Aggregate verwendet werden.

Bevorzugt werden die erfindungsgemässen Gemische als Laminierharze und insbesondere als Klebharze eingesetzt.

Bei den Dimethylaminoderivaten handelt es sich um flüssige bis viskose oder kristalline Substanzen, die schwerflüchtig sind und auch bei Verarbeitung der erfindungsgemässen Gemische nur wenig verdampfen, so dass hierbei kaum Geruchsbelästigungen auftreten. Die erfindungsgemässen härtbaren Gemische weisen überraschend längere Gelierzeiten (also verbesserte Verarbeitungsmöglichkeiten) auf als jene Gemische, die N,N-Dimethyl-1,3-propylendiamin enthalten, wobei ähnlich gute und zum Teil bessere Haftfähigkeiten beim Einsatz als Klebharze erzielt werden.

Die nachfolgenden Beispiele dienen zur näheren Erläuterung der Erfindung:

Herstellungsbeispiele

Beispiel 1

β-Dimethylaminoäthyl-β-cyanoäthyläther

$$(CH_3)_2NCH_2CH_2OCH_2CH_2CN$$

Zu 356,5 g 2-Dimethylaminoäthanol und 5 ml Triton B lässt man 244 g Acrylnitril innerhalb 15 Minuten zutropfen, wobei sich das Gemisch von Raumtemperatur auf 75 °C erwärmt. Danach wird unter weiterem Zusatz von 5 ml Triton B 8 Stunden auf 95 °C erhitzt und anschliessend destilliert. Ausbeute 451,4 g (79,4%) gaschromatographisch einheitliches Produkt; Sdp. 102 °C/8 Torr.

Beispiel 2

γ-Dimethylaminopropyl-β-cyanopropyläther

$$(CH_3)_2N(CH_2)_3OCH_2\underset{\displaystyle CH_3}{\underset{|}{C}}HCN$$

206,2 g 3-Dimethylamino-1-propanol werden wie zuvor mit 161 g Methacrylnitril in Gegenwart von 10 ml Triton B 10 Stunden auf 68° erhitzt. Nach Neutralisation des Katalysators mit 5 ml Eisessig, Extraktion nach dem Auflösen in 300 ml Chloroform mit $H_2O$, dem Einengen und Destillieren erhält man 148,8 g (44,3%) gaschromatographisch zu über 95% einheitliches Produkt vom Sdp. 109 °C/9 Torr.

Beispiel 3

β-Dimethylaminopropyl-(2)-β-cyanoäthyläther

$$(CH_3)_2NCH_2\underset{\displaystyle CH_3}{\underset{|}{C}}HOCH_2CH_2CN$$

Zu 268,0 g 1-Dimethylamino-2-propanol und 22 ml 20%iger Natronlauge lässt man innerhalb 1 Stunde 165 g Acrylnitril bei 55–60 °C zutropfen und danach 1 Stunde bei 55 °C reagieren. Man fügt weitere 5 ml 20%ige NaOH und 42 g Acrylnitril zu, lässt eine weitere Stunde bei 55° reagieren, löst dann alles mit 100 ml Toluol, wäscht mit wenig $H_2O$, engt ein und destilliert. Ausbeute 326,2 g (80,3%), Sdp. 103 °C/10 Torr.

Beispiel 4

β-Dimethylaminoäthyl-γ-aminopropyläther

$$(CH_3)_2NCH_2CH_2OCH_2CH_2CH_2NH_2$$

156,4 g β-Dimethylaminoäthyl-β-cyanoäthyläther werden in Gegenwart von 550 ml Äthanol, 150 g gasförmigem Ammoniak und 8 g Raney-Nikkel im Autoclav bei 120 °C und einem Druck von 135 atü hydriert. Nach 30 Minuten ist Druckkonstanz erreicht. Die Destillation der vom Katalysator befreiten Lösung gibt 141,9 g (88,2%) Produkt vom Sdp. 78 °C/10 Torr.

Beispiel 5
γ-Dimethylaminopropyl-γ-aminoisobutyläther

$$(CH_3)_2NCH_2CH_2CH_2O\ CH_2\underset{\displaystyle CH_3}{\underset{|}{C}}HCH_2NH_2$$

102 g γ-Dimethylaminopropyl-β-cyanopropyläther werden wie in Beispiel 4 in Gegenwart von 350 ml Äthanol, 100 g $NH_3$ und 8 g Raney-Nickel bei 120 °C und 120 atü hydriert. Die Destillation ergibt 86,4 g (82,7%) Produkt vom Sdp. 95 °C/8 Torr, das gaschromatographisch einheitlich ist.

Beispiel 6
β-Dimethylaminopropyl-(2)-γ-aminopropyläther

$$(CH_3)_2NCH_2\underset{\displaystyle CH_3}{\underset{|}{C}}HO(CH_2)_3NH_2$$

157,8 g β-Dimethylaminopropyl-(2)-β-cyanoäthyläther werden wie in Beispiel 4 in Gegenwart von 250 ml Isopropanol, 160 g $NH_3$ und 15 g Raney-Nickel bei 105 °C und 110 atü hydriert. Die Destillation ergibt 151,7 g (94,7%) gaschromatographisch einheitliches Produkt vom Sdp. 82 °C/11 Torr.

Beispiel 7
Essigsäure-β-dimethylaminoäthylester

$$(CH_3)_2NCH_2CH_2OCOCH_3$$

Zu 133,6 g 2-Dimethylaminoäthanol lässt man innerhalb 20 Minuten unter leichter Kühlung 168,5 g Essigsäureanhydrid zutropfen. Die Temperatur steigt dabei von 20 °C auf maximal 105 °C. Man hält 30 Minuten auf 100 °C, lässt erkalten, fügt 750 ml $CHCl_3$ zu und neutralisiert die entstandene Essigsäure mit 380 ml 20%iger NaOH-Lösung unter Kühlung bei 30–35 °C. Man trennt die beiden Phasen, wäscht die organische Phase mit wenig $H_2O$, engt ein und destilliert den Rückstand. Ausbeute 151,7 g (77,7%); Sdp. 114 °C/225 Torr.

Beispiel 8
Essigsäure-β-dimethylaminopropylester

$$(CH_3)_2N(CH_2)_3OCOCH_3$$

14,4 g 3-Dimethylamino-1-propanol und 14,3 g Essigsäureanhydrid werden gemäss Beispiel 7 umgesetzt und aufgearbeitet. Ausbeute 14,9 g (73,1%), Sdp. 53 °C/12 Torr. Das Produkt ist gaschromatographisch einheitlich.

Beispiel 9
Propionsäure-γ-dimethylaminopropylester

$$(CH_3)_2N(CH_2)_3OCOC_2H_5$$

10,8 g 3-Dimethylamino-1-propanol und 12,1 g Propionsäureanhydrid werden gemäss Beispiel 7 umgesetzt und aufgearbeitet. Ausbeute 9,6 g (58,1%), Sdp. 85 °C/20 Torr.

Beispiel 10
Isobuttersäure-(2)-β-dimethylaminopropylester

$$(CH_3)_2NCH_2\underset{\displaystyle CH_3}{\underset{|}{C}}HOCOCH(CH_3)_2$$

Zu 103 g 1-Dimethylamino-2-propanol in 200 ml Chloroform lässt man eine Lösung von 107 g Isobuttersäurechlorid in 150 ml $CHCl_3$ zutropfen. Die Reaktion ist exotherm und das Gemisch siedet von selbst am Rückfluss. Man lässt 45 Minuten nachreagieren, tropft unter Kühlung bei 25–30 °C 210 ml 20%ige NaOH zu, trennt die Phasen, wäscht mit 100 ml $H_2O$ und engt ein. Die Destillation des Rückstands ergibt 127,7 g Produkt (74,0%), Sdp. 72 °C/18 Torr.

Beispiel 11
Laurinsäure-γ-dimethylaminopropylester

$$(CH_3)_2N(CH_2)_3OCO(CH_2)_{10}CH_3$$

Die Darstellung erfolgt gemäss Beispiel 10 aus 72,2 g 3-Dimethylamino-1-propanol und 153,1 g Laurinsäurechlorid in insgesamt 250 ml $CHCl_3$; Neutralisation mit 147 ml 20%iger NaOH. Ausbeute 157,2 g (82,1%); Sdp. 110 °C/0,1 Torr.

Beispiel 12
Benzoesäure-β-dimethylaminoäthylester

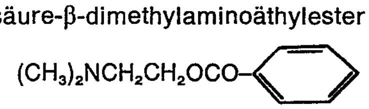

Die Darstellung erfolgt gemäss Beispiel 10 aus 89,1 g 2-Dimethylamino-äthanol und 140,5 g Benzoylchlorid in insgesamt 300 ml $CHCl_3$; Neutralisation mit 210 ml 20%iger NaOH. Ausbeute 187,7 g (97,1%) gaschromatographisch einheitliches Produkt; Sdp. 127 °C/9 Torr.

Beispiel 13
N-Phenylcarbaminsäure-β-dimethylaminoäthylester

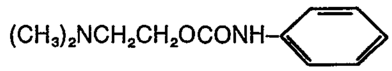

Man lässt zu 96 g Dimethylaminoäthanol unter Kühlung bei 60–70 °C 128 g Phenylisocyanat innerhalb 45 Minuten zutropfen. Nach weiteren 30 Minuten bei 65 °C werden tiefsiedende Anteile bei einem Druck von 0,02 Torr und einer Badtemperatur von 90 °C entfernt. Der Rückstand von 218,4 g (97,6%) ist eine hellgelbe Flüssigkeit und besteht weitgehend aus dem obigen Ester. Titration: 1 Äquiv. = 208,4 g (Theorie 208,2). IR-Spektrum

3320 cm$^{-1}$ NH, 1720 cm $^{-1}$ C = O; zwischen 2000 und 2500 cm$^{-1}$ keine Banden, d.h. Isocyanat ist nicht mehr vorhanden. Kleine Mengen von 5–10 g lassen sich im Kugelrohrofen bei 135 °C/0,015 Torr destillieren; grössere Mengen zersetzen sich bei der Destillation.

Beispiel 14

N-Cyclohexylcarbaminsäure-γ-dimethylaminopropylester

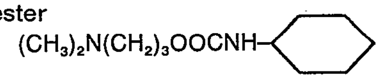

77,3 g 3-Dimethylamino-1-propanol werden gemäss Beispiel 13 mit 94 g Cyclohexylisocyanat umgesetzt. Die Destillation des Reaktionsprodukts ergibt 153,4 g (92,8%) gaschromatographisch einheitliches Produkt vom Sdp. 105 °C/0,015 Torr. IR-Spektrum: 3320 cm$^{-1}$ NH, 1700 cm$^{-1}$ C = O, keine Banden zwischen 2000 und 2500 cm$^{-1}$.

Beispiel 15

N-Butylcarbaminsäure-(2)-β-dimethylaminopropylester

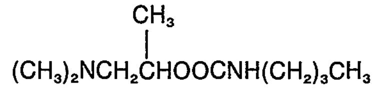

71,8 g 1-Dimethylamino-2-propanol werden gemäss Beispiel 13 mit 69,0 g Butylisocyanat umgesetzt. Die Destillation ergibt 118,5 g (84,2%) Produkt vom Sdp. 72 °C/0,015 Torr. IR-Spektrum: 3340 cm$^{-1}$ NH, 1700 cm$^{-1}$ CO, keine Banden zwischen 2000 und 2500 cm$^{-1}$.

Beispiel 16

Toluylen-2,4-N,N'-bis-(carbaminsäure-β-dimethylaminoäthylester)

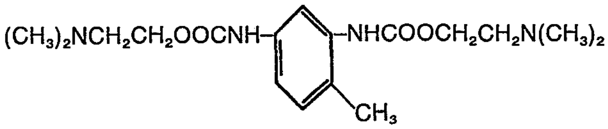

71,3 g 2-Dimethylaminoäthanol und 69,6 g Toluylen-2,4-diisocyanat werden gemäss Beispiel 13 bei 70 °C umgesetzt zu 138,8 g Rohprodukt. 125,1 g werden aus 500 ml Acetonitril umkristallisiert; Ausbeute 96,2 g (75,8%); Smp. 112–113 °C.

Beispiel 17

N-Diäthylcarbaminsäure-β-dimethylaminoäthylester

$(CH_3)_2NCH_2CH_2OOCN(C_2H_5)_2$

Zu 500 ml einer Lösung bestehend aus 99 g Phosgen und Toluol gibt man weitere 500 ml Methylenchlorid und lässt innerhalb einer Stunde eine Lösung von 178 g Dimethylaminoäthanol und 300 g Triäthylamin in 500 ml Methylenchlorid bei 10–20 °C zutropfen. Anschliessend wird 2 Stunden am Rückfluss gekocht, filtriert und das Filtrat eingeengt. Die Destillation im Hochvakuum ergibt 136,5 g (72,5%) Produkt vom Sdp. 68–72 °C/0,01 Torr, dessen NMR-Spektrum obige Struktur bestätigt.

Beispiel 18

N-(n-Butyl)-N'-(β-dimethylaminopropyl)-harnstoff

$(CH_3)_2NCH_2CH_2CH_2NHCONH(CH_2)_3CH_3$

Unter Rühren und Kühlung auf 55–60 °C tropft man zu 51,1 g frisch destilliertem 3-Dimethylamino-1-propylamin unter $N_2$-Atmosphäre 49,5 g frisch destilliertes Butylisocyanat (15 Min.). Nach der Nachreaktion (1 Std. bei 60 °C) werden tiefsiedende Anteile bei einem Druck von 0,1 Torr und einer maximalen Badtemperatur von 140 °C entfernt. Man erhält 98,7 g (98%) Produkt obiger Struktur. Titration: 1 Äquiv. = 208,3 g (Theorie 201,3).

Analyse $C_{10}H_{23}N_3O$ (M = 201,31)

| | | | |
|---|---|---|---|
| ber.: | C 59,66 | H 11,51 | N 20,87 |
| gef.: | C 59,57 | H 11,71 | N 20,57 |

Beispiel 19

N-Phenyl-N'-(β-dimethylaminoäthyl)-harnstoff

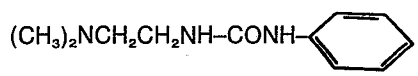

Man löst 88 g Dimethylaminoäthylamin in 500 ml Toluol und tropft innerhalb 10 Minuten 119 g Phenylisocyanat zu, wobei die Temperatur auf 97 °C ansteigt. Man kocht 4 Stunden am Rückfluss; beim Erkalten kristallisiert das Produkt aus. Man saugt ab und kristallisiert das feuchte Produkt aus 1000 ml Essigester um.

Ausbeute 135 g (65,1%); Smp. 141–142 °C.

Beispiel 20

N-(n-Butyl)-N'-(β-cyanopropyl)-N'-(γ-dimethylaminopropyl)-harnstoff

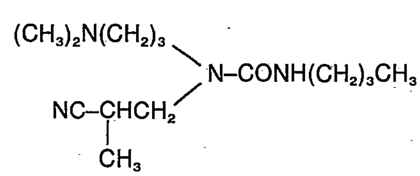

Zu 169,3 g N-(β-cyanopropyl)-dimethylaminopropylamin, gelöst in 500 ml Methylenchlorid, lässt man unter $N_2$-Atmosphäre eine Lösung von 99,1 g Butylisocyanat in 100 ml Methylenchlorid innerhalb 1 Stunde zutropfen. Danach wird 2 Stunden am Rückfluss gekocht, eingeengt und der Rückstand bei 80 °C im Hochvakuum behandelt, um alle tiefsiedenden Anteile zu entfernen. Ausbeute 249,2 g (90,8%) Öl.

Titration: 1 Äquiv. = 261,9 g (Theorie 268,4).

Analyse $C_{14}H_{28}N_4O$ (M = 268,41)

ber.: C 62,65 H 10,52 N 20,88
gef.: C 62,5 H 10,5 N 20,6

Beispiel 21
N,N'-Dimethylaminopropylharnstoff

$$(CH_3)_2N(CH_2)_3NH\text{–}CO\text{–}NH(CH_2)_3N(CH_3)_2$$

Man erhitzt ein Gemisch aus 204 g Dimethylaminopropylamin und 60 g Harnstoff innerhalb 2 Stunden allmählich auf 140 °C und hält über Nacht aud dieser Temperatur. Es entsteht eine klare Lösung, aus der sich $NH_3$-Gas abspaltet. Danach wird weitere 5 Stunden auf 200 °C erhitzt und das Gemisch nach dem Erkalten destilliert. Ausbeute 126,1 g (54,8%) Harnstoff mit obiger Struktur; Sdp. 183–185 °C/0,1 Torr.

Analyse $C_{11}H_{26}N_4O$ (M = 230,36)
ber.: C 57,36 H 11,38 N 24,32
ger.: C 57,2 H 11,3 N 24,2

Beispiel 22
Hexamethylen-N,N-bis-(γ-dimethylamino-propyl)-harnstoff

$$(CH_3)_2N(CH_2)_3NHCONH(CH_2)_6NHCONH(CH_2)_3N(CH_3)_2$$

Zu einer Lösung von 168,2 g Hexamethylendiisocyanat in 1000 ml Methylenchlorid lässt man 204,3 g Dimethylaminopropylamin zutropfen und kocht über Nacht am Rückfluss. Dabei scheiden sich Kristalle ab, die bei Raumtemperatur abgesaugt, mit $CH_2Cl_2$ gewaschen und getrocknet werden. Die Umkristallisation aus einem Gemisch von 1000 ml Aceton und 800 ml Äthanol ergibt 221,0 g (59,3%) Produkt vom Smp. 159 °C. Titration: 2 Äquiv. = 376,7 g (Theorie 372,6).

Beispiel 23
β-Dimethylaminoethylcarbonat

$$[(CH_3)_2NCH_2CH_2O]\text{–}_2CO$$

Mittels einer 80 cm hohen Füllkörperkollone mit gesteuertem Kolonnenkopf wird aus einem Gemisch bestehend aus 118 g Diäthylcarbonat, 178 g 2-Dimethylaminäthanol und 0,66 g KOH langsam Äthanol abdestilliert. Man erhält so 153 g Rohprodukt, dessen Destillation 82,4 g eines Gemisches vom Sdp. 88–120 °C bei 14 Torr ergibt. Die erneute Destillation von 77 g dieses Gemisches über eine Drehbandkolonne ergibt 41,7 g Produkt vom Sdp. 111 °C/14 Torr. Titration: 1 Äquiv. = 101,6 g (Theorie 102,1). Das NMR-Spektrum bestätigt obige Struktur.

Beispiel 24
β-Dimethylaminoäthyl-γ-dimethylaminopropyläther

$$(CH_3)_2NCH_2CH_2OCH_2CH_2CH_2N(CH_3)_2$$

158 g β-Dimethylaminoäthyl-γ-aminopropyläther, hergestellt nach Beispiel 4, werden unter Kühlung 250 g Ameisensäure zugetropft. Danach lässt man innerhalb einer Stunde 187 ml einer 38%igen wässerigen Formaldehydlösung bei 55–65 °C zutropfen, wobei sich $CO_2$ abspaltet. Man erhöht danach die Innentemperatur allmählich auf 116 °C, rührt noch weitere 17 Stunden, tropft sodann 224 g konz. HCl zu und engt am Rotationsverdampfer ein. Nach Zugabe einer Lösung von 125 g NaOH in 400 ml $H_2O$ scheidet sich das Amin als Öl ab. Es wird abgetrennt, und die wässerige Phase wird 4mal mit je 50 ml Toluol extrahiert. Die Destillation der Extrakte und des Amins ergibt 101,4 g (54,2%) reines Amin vom Sdp. 110 °C/13 mbar. Der Vorlauf enthält weitere Mengen des Amins. Das IR-Spektrum des reinen Amins zeigt keine NH-Bande oberhalb 3000 $cm^{-1}$.

Beispiel 25
β-Dimethylaminopropyl-(2)-γ-dimethylamino-propyläther

$$(CH_3)_2NCH_2CHOCH_2CH_2CH_2N(CH_3)_2$$
$$CH_3$$

49,7 g β-Dimethylaminopropyl-(2)-γ-aminopropyläther werden wie im Beispiel 24 unter Verwendung von 71 g Ameisensäure, 54 ml 38%igem Formaldehyd, 64 g konz. HCl, sowie 75 g NaOH gelöst in 300 ml $H_2O$ umgesetzt und aufgearbeitet. Nach der Extraktion des Amins mit Toluol wird destilliert. Ausbeute 41,4 g gaschromatographisch einheitliches Amin vom Sdp. 110 °C/20 mbar.

Beispiel 26
Benzyl-γ-dimethylaminopropyläther

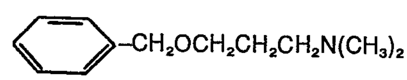

77 g Benzyl-γ-aminopropyläther werden wie im Beispiel 24 unter Verwendung von 107 g Ameisensäure, 80 ml 38%igem Formaldehyd, 48 g konz. HCl, sowie 35 g NaOH gelöst in 350 ml $H_2O$ umgesetzt und aufgearbeitet. Nach der Extraktion des Amins wird destilliert. Ausbeute 55,6 g (61,8%) Reinamin vom Sdp. 134 °C/20 mbar. Das IR-Spektrum zeigt keine NH-Bande oberhalb 3000 $cm^{-1}$.

Beispiel 27
Cyclohexyl-γ-dimethylaminopropyläther

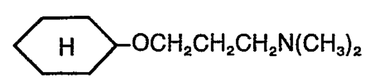

180 g Cyclohexyl-γ-aminopropyläther werden wie im Beispiel 24 unter Verwendung von 250 g Ameisensäure, 190 ml 38%igem Formaldehyd, 113 g konz. HCl, sowie 66 g NaOH gelöst in 200 ml $H_2O$ umgesetzt und aufgearbeitet. Die Destillation ergibt 121,5 g (60,2%) gaschromatographisch einheitliches Amin vom Smp. 115 °C/18 mbar.

Beispiel 28
Dodecyl-γ-dimethylaminopropyläther

$$CH_3(CH_2)_{11}OCH_2CH_2CH_2N(CH_3)_2$$

191 g Dodecyl-$\gamma$-aminopropyläther werden wie im Beispiel 24 unter Verwendung von 180 g Ameisensäure, 136 ml 38%igem Formaldehyd, 81 g konz. HCl, sowie 98 g NaOH gelöst in 500 ml $H_2O$ umgesetzt und aufgearbeitet. Zur Extraktion des Amins wird ein Gemisch aus Butanol und Hexan verwendet. Die Destillation ergibt 99,6 g (48,4%) gaschromatographisch einheitliches Amin vom Sdp. 130 °C/0,06 mbar.

Beispiel 29

Undecyl-(2)-$\gamma$-dimethylaminopropyläther

$$CH_3(CH_2)_8\underset{\displaystyle CH_3}{\underset{|}{C}}HOCH_2CH_2CH_2N(CH_3)_2$$

70,3 g Undecyl-(2)-$\gamma$-aminopropyläther werden wie im Beispiel 24 unter Verwendung von 70 g Ameisensäure, 53 ml 38%igem Formaldehyd, 32 g konz. HCl, sowie 23 g NaOH gelöst in 350 ml $H_2O$ umgesetzt und aufgearbeitet. Die Extraktion des Amins erfolgt mit 3 Portionen Butanol zu je 100 ml. Die Destillation ergibt 37,8 g (47,9%) Amin vom Sdp. 110 °C/0,06 mbar.

Beispiel 30

1,4-Cyclohexyl-di-($\gamma$-dimethylaminopropyl)-äther

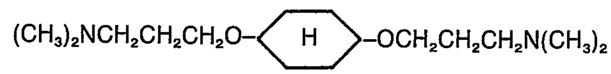

66,1 g 1,4-Cyclohexyl-di-($\gamma$-aminopropyl)-äther, der infolge unvollständiger Cyanoäthylierung und Hydrierung eine kleinere Menge 4-Hydroxycyclohexyl-$\gamma$-aminopropyläther enthält, werden wie im Beispiel 24 unter Verwendung von 132 g Ameisensäure, 100 ml 38%igem Formaldehyd, 60 g konz. HCl, sowie 38 g NaOH gelöst in 250 ml $H_2O$ umgesetzt und aufgearbeitet. Man erhält nach dem Einengen 77 g Rohamin. Die Destillation über eine Füllkörperkolonne ergibt ein gaschromatographisch einheitliches Diamin vom Sdp. 89 °C/ 0,005 mbar.

Beispiel 31

Terephthalsäure-di-($\gamma$-dimethylaminopropyl)-ester

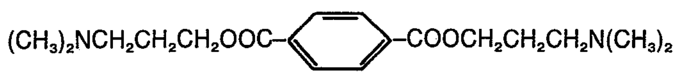

Zu einer Lösung von 81,9 g 3-Dimethylamino-1-propanol in 450 ml Chloroform lässt man bei 45–50 °C eine Lösung von 61 g Terephthalsäurechlorid in 200 ml $CHCl_3$ zutropfen, wobei sich Kristalle abscheiden. Nach 15 Minuten Nachreaktion werden bei 25° Innentemperatur 132 ml einer 20%igen NaOH-Lösung zugetropft, wobei sich die Kristalle auflösen. Nach dem Abtrennen der organischen Phase, dem Einengen und Destillieren erhält man 65,4 g reines Amin (65,5%) vom Sdp. 157 °C/0,04 mbar.

Beispiel 32

Adipinsäure-di-($\beta$-dimethylaminoäthyl)-ester

$$(CH_3)_2NCH_2CH_2OOC–(CH_2)_4COOCH_2CH_2N(CH_3)_2$$

Zu 89 g Dimethylaminoäthanol in 400 ml $CHCl_3$ lässt man 91,5 g Adipinsäuredichlorid gelöst in 400 ml $CHCl_3$ bei 45–50 °C zutropfen. Danach lässt man 220 ml einer 20%igen NaOH-Lösung bei 25 °C zutropfen und trennt die organische Phase ab. Extraktion mit 3mal 100 ml $CHCl_3$ und Einengen ergibt 141 g Rohprodukt und nach der Destillation 76,3 g (53%) Reinprodukt vom Sdp. 160 °C/ 0,03 mbar.

Beispiel 33

Dodecandisäure-di-($\beta$-dimethylaminopropyl)-ester

$$(CH_3)_2NCH_2\overset{\displaystyle CH_3}{\overset{|}{C}}HOOC–(CH_2)_{10}–COO\overset{\displaystyle CH_3}{\overset{|}{C}}HCH_2N(CH_3)_2$$

Zu 51,6 g 3-Dimethylamino-2-propanol in 250 ml $CHCl_3$ lässt man 66,8 g Dodecandisäurechlorid gelöst in 350 ml $CHCl_3$ bei 45–50° zutropfen. Danach werden 110 ml 20%ige NaOH bei 25 °C zugetropft, die organische Phase wird abgetrennt, die wässerige Phase 2mal mit je 100 ml $CHCl_3$ extrahiert und alle organischen Phasen werden zusammen eingeengt (97,4 g). Die Destillation von 92 g ergibt 50,6 g (53,5%) gaschromatographisch einheitliches Amin vom Sdp. 163 °C/0,03 mbar.

B. Anwendungsbeispiel I

Die in nachfolgender Tabelle I aufgeführten Dimethylaminoderivate werden mit einem flüssigen Epoxidharz auf Basis von Bisphenol-A und Epichlorhydrin (Epoxidgehalt 5,4 Val/kg; Viskosität nach DIN 53015 bei 25 °C 10 500 -$^{mPaS}$) vermischt. Der Gehalt an Dimethylaminoderivaten ist in Gew.-Teilen auf 100 Gew.-Teile Epoxidharz angegeben.

Die Klebefestigkeit wird mittels Messung der Zugscherfestigkeit nach DIN 53 283 an geschliffenen und entfetteten Antikorrodal-100-B-Proben mit 12 mm Überlappung bestimmt. Härtungszeit: 3 Stunden bei 100 °C.

Die Gelierzeit wird bei 120 °C mit Probemengen von etwa ½ g auf einer thermostatisierten Heizplatte bestimmt.

Tabelle I

| Dimethylaminoderivat | Menge | Gelierzeit Min. | Gelierzeit sec | Zugscherfestigkeit ($N/mm^2$) |
|---|---|---|---|---|
| $D(CH_2)_2O(CH_2)_3NH_2$ | 14,3 | 2 | 40 | 12,9 |
| $D(CH_2)_2O(CH_2)_2CN$ | 16 | 2 | 55 | 21,0 |
| $D(CH_2)_3OCH_2CH(CH_3)CN$ | 19,2 | 4 | 30 | 15,8 |
| $D(CH_2)_3OCH_2CH(CH_3)CH_2NH_2$ | 17,0 | 2 | 45 | 18,7 |
| $D(H_2CC(CH_3)_2CH_2O(CH_2)_2CN$ | 15,5 | > 210 Min. | | 12,4 |
| $DCH_2CH(CH_3)O(CH_2)_2CN$ | 17,6 | 10 | 0 | 16,1 |
| $DCH_2CH(CH_3)O(CH_2)_3NH_2$ | 15,7 | 4 | 40 | 18,3 |
| 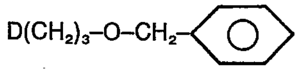 | 21,6 | 8 | 50 | 19,2 |
| 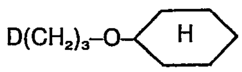 | 20,8 | 7 | 25 | 18,5 |
| 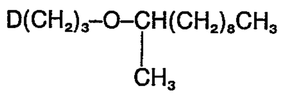 | 29,3 | 33 | 15 | 14,2 |
| 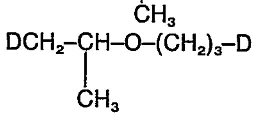 | 10,6 | 6 | 35 | 14,3 |
| $D(CH_2)_2-O-(CH_2)_3-D$ | 9,8 | 5 | 55 | 16,3 |
| 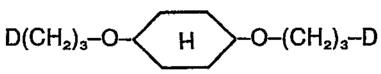 | 16,1 | 7 | – | 14,4 |
| $D(CH_2)_2OCOCH_3$ | 14,8 | 15 | 50 | 17,9 |
| $D(CH_2)_3OCOCH_3$ | 14,2 | 8 | 50 | 20,9 |
| $D(CH_2)_3OCOCH_3$ | 10,7 | 10 | 40 | 19,0 |
| $D(CH_2)_3OCOC_2H_5$ | 15,6 | 10 | 10 | 21,2 |
| $D(CH_2)_3OCOC_2H_5$ | 11,7 | 8 | 50 | 18,8 |
| $D(CH_2)_3OCOn-C_{11}H_{23}$ | 32,2 | 19 | 0 | 23,0 |
| $DCH_2CH(CH_3)OCOCH(CH_3)_2$ | 19,5 | 46 | 0 | 19,9 |
| $D(CH_2)_2OCOC_6H_5$ | 16,4 | 15 | 0 | 23,2 |
| 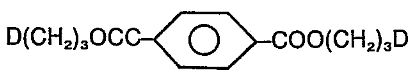 | 19,0 | 6 | 25 | 18,8 |
| 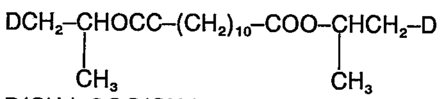 | 22,5 | 18 | 05 | 19,8 |
| $D(CH_2)_2OOC(CH_2)_4-COO(CH_2)_2D$ | 16,3 | 10 | 30 | 22,0 |
| $D(CH_2)_2NHCONHC_6H_5$ | 23,4 | 2 | 55 | 15,5 |
| 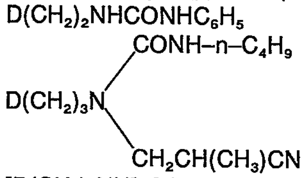 | 30,2 | 12 | 0 | 16,2 |
| $[D(CH_2)_3NH]_2CO$ | 13,0 | 2 | 15 | 12,6 |
| $[D(CH_2)_3NHCONH]_2(CH_2)_6$ | 15,8 | 2 | 40 | 14,5 |
| $D(CH_2)_2OCON(C_2H_5)_2$ | 13,8 | 25 | 20 | 18,0 |
| $D(CH_2)_2OCONHC_6H_5$ | 17,6 | 8 | 50 | 19,5 |
| $D(CH_2)_3OCONHC_6H_{11}$ | 25,7 | 7 | 30 | 19,0 |
| $DCH_2CH(CH_3)OCONHC_4H_9$ | 22,8 | 19 | 0 | 23,1 |
| 2,4–Toluylen$[NHCOO(CH_2)_3D]_2$ | 14,9 | 7 | 20 | 14,7 |
| $[D(CH_2)_2O]_2CO$ | 11,5 | 8 | 20 | 16,7 |
| | 12,5 | 1 | 35 | 16,4 |
| $D(CH_2)_3NH_2$ | 10 | 1 | 50 | 15,0 |
| (Vergleich) | 5 | 3 | 25 | 12,6 |

D bedeutet in der Tabelle die $(CH_3)_2N$-Gruppe.

Anwendungsbeispiel II

Die in der nachfolgenden Tabelle II aufgeführten Dimethylaminoderivate werden mit einem flüssigen Epoxid-Phenol-Novolakharz, mit einem Epoxidgehalt von 5,68 Val/kg vermischt. Der Gehalt an Dimethylaminoderivaten ist den Gew.-Teilen auf 100 Gew.-Teile Epoxidharz angegeben.

Die Prüfbedingungen zur Prüfung der Klebefestigkeit sowie der Gelierzeit sind analog Anwendungsbeispiel I.

Tabelle II

| Dimethylaminoderivat | Menge | Gelierzeit Min. | sec | Zugscherfestigkeit (N/mm²) |
|---|---|---|---|---|
| D–$(CH_2)_3$–$NH_2$ (Vergleich) | 10,5 | – | 55 | 10,7 |
| 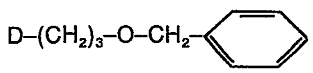 | 22,7 | 3 | 20 | 12,1 |
| 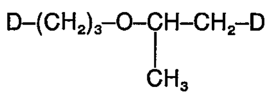 | 11,1 | 2 | 45 | 9,0 |
| 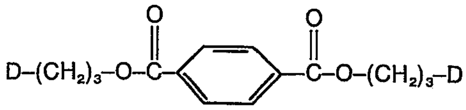 | 20,0 | 3 | 20 | 15,8 |
| 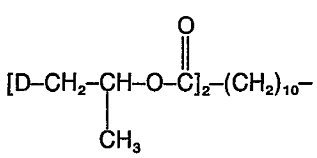 | 23,7 | 11 | 25 | 13,3 |

Anwendungsbeispiel III

Die in nachfolgender Tabelle III aufgeführten Dimethylaminoderivate werden mit dem flüssigen Tetrahydrophthalsäurediglycidylester techn. Qualität (Epoxidgehalt 6,1 Val/kg., Viskosität 600 mPa · s/25 °C) vermischt.

Der Gehalt an Dimethylaminoderivaten ist in Gew.-Teilen auf 100 Gew.-Teile Epoxidharz angegeben.

Die Prüfbedingungen zur Prüfung der Klebefestigkeit sowie der Gelierzeit sind analog Anwendungsbeispiel I.

Tabelle III

| Dimethylaminoderivat | Menge | Gelierzeit Min. | sec | Zugscherfestigkeit (N/mm²) |
|---|---|---|---|---|
| D–$(CH_2)_3$–$NH_2$ (Vergleich) | 11,3 | 3 | – | 13,2 |
| 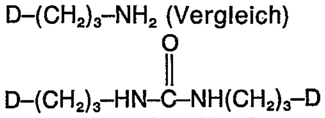 | 14,7 | 4 | – | 15,0 |
| 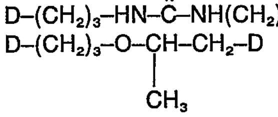 | 12,0 | 3 | 50 | 10,8 |
| 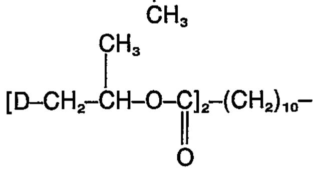 | 25,4 | 17 | 20 | 12,4 |

Anwendungsbeispiel IV

Die in nachfolgender Tabelle IV aufgeführten Dimethylaminoderivate werden mit einem flüssigen Hydantoindiglycidyläther techn. Qualität (Epoxidgehalt 7,2 Val/kg; Wirkstoff 1250 mPa . s/25 °C) vermischt.

Der Gehalt an Dimethylaminoderivaten ist in Gew.-Teilen auf 100 Gew.-Teile Epoxidharz angegeben.

Die Prüfbedingungen zur Prüfung der Klebefestigkeit sowie der Gelierzeit sind analog Anwendungsbeispiel I.

Tabelle IV

| Dimethylaminoderivat | Menge | Gelierzeit Min. | sec | Zugscherfestigkeit ($N/mm^2$) |
|---|---|---|---|---|
| $D-(CH_2)_3-NH_2$ (Vergleich) | 13,3 | 1 | 9 | 11,3 |
| $D-(CH_2)_2-O-(CH_2)_2CN$ | 21,3 | 2 | 25 | 10,3 |
| 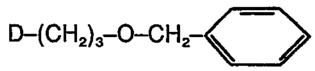 | 28,8 | 9 | 20 | 8,9 |
| $D-(CH_2)_3-O-\underset{CH_3}{\underset{\|}{C}}H-CH_2-D$ | 14,1 | 4 | 45 | 9,9 |
| $D-(CH_2)_2-O-\overset{O}{\overset{\|}{C}}-CH_3$ | 19,7 | 22 | - | 12,2 |
| 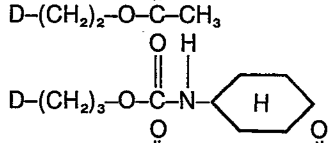 | 34,3 | 10 | - | 12,2 |
| 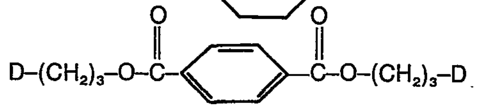 | 25,3 | 7 | 45 | 14,8 |
| $[D-CH_2-\overset{CH_3}{\overset{\|}{C}}H-O-\overset{O}{\overset{\|}{C}}]_2-(CH_2)_{10}-$ | 30,0 | 36 | 40 | 14,9 |

**Patentansprüche**

1. Härtbare Gemische, enthaltend (a) eine Polyepoxidverbindung mit durchschnittlich mehr als einer Epoxidgruppe im Molekül und (b) einen Härter, dadurch gekennzeichnet, dass der Härter ein Dimethylaminoderivat der Formel I

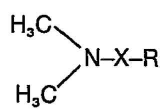 (I)

worin

X 1,3-Propylen oder Äthylen oder durch Alkyl substituiertes Äthylen ist und

R den Formeln

$(CH_3)_2N-X-O\overset{O}{\overset{\|}{C}}O$ (II), $R^1\overset{O}{\overset{\|}{C}}O$ (III), $(CH_3)_2N-X-O\overset{O}{\overset{\|}{C}}Y\overset{O}{\overset{\|}{C}}O$ (IV), $R^2R^3N\overset{O}{\overset{\|}{C}}O$ (V), $(CH_3)_2N-X-O\overset{O}{\overset{\|}{C}}NH-Y-HN\overset{O}{\overset{\|}{C}}O$ (VI),

$OR^4$ (VII) oder $OR^{11}OXN(CH_3)_2$ (XI) entspricht, worin $R^1$ Alkyl, Cycloalkyl, unsubstituiertes oder mit Halogen oder $C_1$–$C_4$-Alkyl substituiertes Aryl oder Aralkyl bedeutet, Y eine direkte Bindung, Alkylen, Alkenylen, Cycloalkylen oder Arylen ist, $R^2$ ein Wasserstoffatom ist oder die Bedeutung von $R^3$ hat und $R^3$ Alkyl, Cycloalkyl oder mit $C_1$–$C_4$-Alkyl oder Halogen substituiertes oder unsubstituiertes Aryl oder Aralkyl darstellt, und $R^4$ Alkyl, Alkenyl, Cycloalkyl, Cycloalkylalkylen, unsubstituiertes oder mit $C_1$–$C_4$-Alkyl oder Halogen substituiertes Aryl oder Aralkyl, $CH_2\underset{R^5}{\underset{|}{C}}H-Z$ oder $\underset{R^5}{\underset{|}{C}}H-CH_2-Z$ ist,

worin $R^5$ ein Wasserstoffatom oder Methyl und Z CN, $CH_2NH_2$ oder $CH_2N(CH_3)_2$ bedeutet, und wobei X zusätzlich Neopentylen ist, wenn R den Rest $OR^4$ darstellt, $R^{11}$ Alkylen, Cycloalkylen oder Arylen ist, oder

X Äthylen, 1,3-Propylen oder in 1- oder 2-Stellung durch Methyl substituiertes 1,3-Propylen ist und

R den Formeln $NR^6\overset{O}{\overset{\|}{C}}OR^7$ (VIII), $R^8R^9N\overset{O}{\overset{\|}{C}}NR^{10}$ (IX)

oder $(CH_3)_2N-X-NH\overset{O}{\overset{\|}{C}}NH-(Y-NH\overset{O}{\overset{\|}{C}}NH)_n$ (X) entspricht, worin X und Y unabhängig die zuvor angegebene Bedeutung haben, n 0 oder 1 ist, $R^6$ ein Wasserstoffatom, Alkyl, Cycloalkyl, β-Cyanoäthyl oder in 1- oder 2-Stellung durch Methyl substituiertes β-Cyanoäthyl ist, $R^7$ Alkyl, Alkenyl, Cycloalkyl, Aryl, Aralkyl, Alkaryl oder Alkaralkyl bedeutet, $R^8$ und $R^{10}$ ein Wasserstoffatom und $R^9$ Alkyl, Cycloalkyl, Aryl, Aralkyl, Alkaryl, Alkaralkyl, 3-(Dimethylamino)-propyl oder $R^8$ und $R^9$ unabhängig voneinander $C_1$–$C_4$-Alkyl oder Phenyl und $R^{10}$ ein Wasserstoffatom oder $R^8$ ein Wasserstoffatom, $R^9$ Alkyl, Cycloalkyl, Aryl, Aralkyl, Alkaryl oder Aralkaryl und $R^{10}$ β-Cyanoäthyl oder in 1- oder 2-Stellung durch Methyl substituiertes β-Cyanoäthyl sind, ist.

2. Gemische gemäss Anspruch 1, dadurch gekennzeichnet, dass X Äthylen oder durch $C_1$–$C_{12}$-Alkyl substituiertes Äthylen ist und R den Formeln II–VII entspricht.

3. Gemische gemäss Anspruch 1, dadurch gekennzeichnet, dass X in Formel I Äthylen oder 1,3-Propylen ist und R den Formeln VIII bis X entspricht.

4. Gemische gemäss Anspruch 1, dadurch gekennzeichnet, dass $R^1$ in Formel III $C_1$–$C_{12}$-Alkyl,

$C_5$–$C_7$-Cycloalkyl, unsubstituiertes oder substituiertes Phenyl oder Benzyl ist.

5. Gemische gemäss Anspruch 1, dadurch gekennzeichnet, dass $R^2$ ein Wasserstoffatom und $R^3$ $C_1$–$C_{12}$-Alkyl, $C_5$–$C_7$-Cycloalkyl, unsubstituiertes oder substituiertes Phenyl oder Benzyl oder $R^2$ und $R^3$ $C_1$–$C_4$-Alkyl, $C_5$–$C_7$-Cycloalkyl oder Phenyl bedeuten.

6. Gemische gemäss Anspruch 1, dadurch gekennzeichnet, dass $R^4$ $C_1$–$C_{18}$-Alkyl oder Alkenyl, bevorzugt $C_1$–$C_{12}$-Alkyl oder -Alkenyl, $C_5$–$C_8$-Cycloalkyl, $C_6$–$C_{16}$-Aryl oder $C_7$–$C_{16}$-Aralkyl, β-Nitriläthyl, γ-Aminopropyl oder γ-Dimethylaminopropyl ist.

7. Gemische gemäss Anspruch 1, dadurch gekennzeichnet, dass Y $C_1$–$C_{12}$-Alkylen, $C_5$–$C_7$-Cycloalkylen oder $C_6$–$C_{12}$-Arylen bedeutet.

8. Gemische gemäss Anspruch 1, dadurch gekennzeichnet, dass $R^6$ in seiner Bedeutung als Alkyl 1–4 C-Atome und als Cycloalkyl 6 C-Atome enthält.

9. Gemische gemäss Anspruch 1, dadurch gekennzeichnet, dass $R^7$ Alkyl oder Alkenyl mit 1 bis 18, vorzugsweise 1 bis 12 C-Atomen, $C_5$–$C_7$-Cycloalkyl, $C_6$–$C_{12}$-Aryl, $C_7$–$C_{16}$-Aralkyl oder -Alkaryl oder $C_8$–$C_{16}$-Alkaralkyl ist.

10. Gemische gemäss Anspruch 1, dadurch gekennzeichnet, dass $R^8$ und $R^{10}$ ein Wasserstoffatom und $R^9$ $C_1$–$C_{12}$-Alkyl, $C_5$–$C_7$-Cycloalkyl, $C_6$–$C_{12}$-Aryl, $C_7$–$C_{16}$-Alkaryl oder Aralkyl oder $C_8$–$C_{16}$-Alkaralkyl sind.

11. Gemische gemäss Anspruch 1, dadurch gekennzeichnet, dass $R^8$ ein Wasserstoffatom, $R^9$ $C_1$–$C_{12}$-Alkyl, $C_5$–$C_7$-Cycloalkyl, $C_6$–$C_{12}$-Aryl, $C_7$–$C_{16}$-Alkaryl oder -Aralkyl oder $C_8$–$C_{16}$-Alkaralkyl und $R^{10}$ β-Cyanoäthyl oder in 1- oder 2-Stellung durch Methyl substituiertes β-Cyanäthyl sind.

12. Gemische gemäss Anspruch 1, dadurch gekennzeichnet, dass das Dimethylaminoderivat der Formel I
$(CH_3)_2NCH_2CH_2OCH_2CH_2CN$,
$(CH_3)_2NCH_2CH_2OCOCH_3$,
$(CH_3)_2NCH_2CH_2CH_2NHCONHC_6H_5$,
$(CH_3)_2NCH_2CH_2OCON(C_2H_5)_2$
oder
$[(CH_3)_2NCH_2CH_2O]_2CO$ ist.

**Claims**

1. A curable mixture containing a) a polyepoxide compound containing on average more than one epoxy group in the molecule and b) a curing agent which is a dimethylamino derivative of the formula I

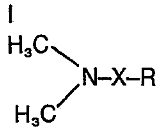

(I)

wherein

X is 1,3-propylene or ethylene or alkyl-substituted ethylene, and

R is a radical of the formula $(CH_3)_2N{-}X{-}O\overset{\overset{O}{\|}}{C}$ (II), $R^1\overset{\overset{O}{\|}}{C}$ (III), $(CH_3)_2N{-}X{-}O\overset{\overset{O}{\|}}{C}Y\overset{\overset{O}{\|}}{C}$ (IV), $R^2R^3N\overset{\overset{O}{\|}}{C}$ (V), $(CH_3)_2N{-}X{-}O\overset{\overset{O}{\|}}{C}NH{-}Y{-}HN\overset{\overset{O}{\|}}{C}$ (VI), $OR^4$ (VII) or $OR^{11}OXN(CH_3)_2$ (XI),

wherein $R^1$ is alkyl, cycloalkyl, or aryl or aralkyl which is unsubstituted or substitued by halogen or $C_1$–$C_4$-alkyl, Y is a direct bond, alkylene, alkenylene, cycloalkylene or arylene, $R^2$ is a hydrogen atom or has the meaning of $R^3$, and $R^3$ is alkyl, cycloalkyl, or aryl or aralkyl which is unsubstituted or substituted by $C_1$–$C_4$-alkyl or halogen, $R^4$ is alkyl, alkenyl, cycloalkyl, cycloalkylalkylene, or aryl or aralkyl which is unsubstituted or substituted by $C_1$–$C_4$-alkyl or halogen, or is $CH_2{-}\underset{\underset{R^5}{|}}{C}H{-}Z$ or $\underset{\underset{R^5}{|}}{C}H{-}CH_2{-}Z$, wherein $R^5$ is a hydrogen atom or methyl and Z is CN, $CH_2NH_2$ or $CH_2N(CH_3)_2$, and $R^{11}$ ist alkylene, cycloalkylene or arylene, and X is additionally neopentylene if R is the radical $OR^4$, or

X is ethylene, 1,3-propylene or 1,3-propylene which is substituted in the 1- or 2-position by methyl, and

R is a radical of the formula

$NR^6\overset{\overset{O}{\|}}{C}OR^7$ (VIII), $R^8R^9N\overset{\overset{O}{\|}}{C}NR^{10}$ (IX)

or $(CH_3)_2N{-}X{-}NH\overset{\overset{O}{\|}}{C}NH{-}(Y{-}NH\overset{\overset{O}{\|}}{C}NH)_n$ (X),

wherein each of X and Y independently is as defined above, n is 0 or 1, $R^6$ is a hydrogen atom, alkyl, cycloalkyl, β-cyanoethyl or β-cyanoethyl which is substituted in the 1- or 2-position by methyl, $R^7$ is alkyl, alkenyl, cycloalkyl, aryl, aralkyl, alkaryl or alkaralkyl, each of $R^8$ and $R^{10}$ is a hydrogen atom and $R^9$ is alkyl, cycloalkyl, aryl, aralkyl, alkaryl, alkaralkyl, 3-(dimethylamino)-propyl, or each of $R^8$ and $R^9$ independently is $C_1$–$C_4$-alkyl or phenyl and $R^{10}$ is a hydrogen atom or $R^8$ is a hydrogen atom, $R^9$ is alkyl, cycloalkyl, aryl, aralkyl, alkaryl or aralkaryl, and $R^{10}$ is β-cyanoethyl or β-cyanoethyl which is substituted in the 1- or 2-position by methyl.

2. A mixture according to claim 1, wherein X is ethylene or ethylene which is substituted by $C_1$–$C_4$-alkyl and R is a radical of the formulae II to VII.

3. A mixture according to claim 1, wherein X in formula I is ethylene or 1,3-propylene and R is a radical of the formulae VIII to X.

4. A mixture according to claim 1, wherein $R^1$ in formula III is $C_1$–$C_{12}$-alkyl, $C_5$–$C_7$-cycloalkyl, or unsubstituted or substituted phenyl or benzyl.

5. A mixture according to claim 1, wherein $R^2$ is a hydrogen atom and $R^3$ is $C_1$–$C_{12}$-alkyl, $C_5$–$C_7$-cycloalkyl, unsubstituted or substituted phenyl or benzyl, or $R^2$ and $R^3$ are $C_1$–$C_4$-alkyl, $C_5$–$C_7$-cycloalkyl or phenyl.

6. A mixture according to claim 1, wherein $R^4$ is alkyl or alkenyl of 1 to 18, preferably 1 to 12, carbon atoms, $C_5$–$C_8$-cycloalkyl, $C_6$–$C_{16}$-aryl or

$C_7$–$C_{16}$-aralkyl, β-cyanoethyl, γ-aminopropyl or γ-dimethylaminopropyl.

7. A mixture according to claim 1, wherein Y is $C_1$–$C_{12}$-alkylene, $C_5$–$C_7$-cycloalkylene or $C_6$–$C_{12}$-arylene.

8. A mixture according to claim 1, wherein $R^6$ as alkyl contains 1 to 4 carbon atoms and as cycloalkyl contains 6 carbon atoms.

9. A mixture according to claim 1, wherein $R^7$ is alkyl or alkenyl of 1 to 18, preferably 1 to 12, carbon atoms, $C_5$–$C_7$-cycloalkyl, $C_6$–$C_{12}$-aryl, $C_7$–$C_{16}$-aralkyl or $C_7$–$C_{16}$-alkaryl, or $C_8$–$C_{16}$-alkaralkyl.

10. A mixture according to claim 1, wherein each of $R^8$ and $R^{10}$ is a hydrogen atom, $R^9$ is $C_1$–$C_{12}$-alkyl, $C_5$–$C_7$-cycloalkyl, $C_6$–$C_{12}$-aryl, $C_7$–$C_{16}$-alkaryl or $C_7$–$C_{16}$-aralkyl, or $C_8$–$C_{16}$-aralkyl.

11. A mixture according to claim 1, wherein $R^8$ is a hydrogen atom, $R^9$ is $C_1$–$C_{12}$-alkyl, $C_5$–$C_7$-cycloalkyl, $C_6$–$C_{12}$-aryl, $C_7$–$C_{16}$-alkaryl or $C_7$–$C_{16}$-aralkyl, or $C_8$–$C_{16}$-alkaralkyl, and $R^{10}$ is β-cyanoethyl or β-cyanoethyl which is substituted in the 1- or 2-position by methyl.

12. A mixture according to claim 1, wherein the dimethylamino derivative of the formula I is:
$(CH_3)_2NCH_2CH_2OCH_2CH_2CN$,
$(CH_3)_2NCH_2CH_2OCOCH_3$,
$(CH_3)_2NCH_2CH_2CH_2NHCONHC_6H_5$,
$(CH_3)_2NCH_2CH_2OCON(C_2H_5)_2$
or
$[(CH_3)_2NCH_2CH_2O]_2CO$.

**Revendications**

1. Mélanges durcissables contenant (a) un composé polyépoxydique à plus d'un radical époxy en moyenne par molécule, et (b) un durcisseur, mélanges caractérisés en ce que le durcisseur est un composé diméthylaminé répondant à la formule I:

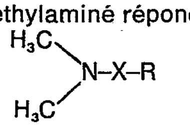 (I)

dans laquelle

X représente un radical propylène-1,3, un radical éthylène ou un radical éthylène porteur d'un alkyle, et

R représente un radical répondant à l'une des formules suivantes:

$(CH_3)_2N{-}X{-}O\overset{O}{\overset{\|}{C}}O$ (II), $R^1\overset{O}{\overset{\|}{C}}O$ (III), $(CH_3)_2N{-}X{-}O\overset{O}{\overset{\|}{C}}Y\overset{O}{\overset{\|}{C}}O$ (IV),

$R^2R^3N\overset{O}{\overset{\|}{C}}O$ (V), $(CH_3)_2N{-}X{-}O\overset{O}{\overset{\|}{C}}NH{-}Y{-}HN\overset{O}{\overset{\|}{C}}O$ (VI),

$OR^4$ (VII) et $OR^{11}OXN(CH_3)_2$ (XI)

dans lesquelles $R^1$ représente un alkyle, un cycloalkyle ou un radical aryle ou aralkyle non substitué ou porteur d'un halogène ou d'un alkyle en $C_1$–$C_4$, Y représente une liaison directe, un alkylène, un alcénylène, un cycloalkylène ou un arylène, $R^2$ représente un atome d'hydrogène ou a la signification de $R^3$, lequel représente un alkyle, un cycloalkyle ou un radical aryle ou aralkyle non substitué ou porteur d'un alkyle en $C_1$–$C_4$ ou d'un halogène, $R^4$ représente un alkyle, un alcényle, un cycloalkyle, un cycloalkyl-alkylène, un radical aryle ou aralkyle non substitué ou porteur d'un alkyle en $C_1$–$C_4$ ou d'un halogène, un radical $-CH_2\underset{R^5}{CH}-Z$ ou un radical $-\underset{R^5}{CH}-CH_2-Z$ (où $R^5$ représente un atome d'hydrogène ou un méthyle et Z représente un radical CN, $CH_2NH_2$ ou $CH_2N(CH_3)_2$), le symbole X pouvant en outre représenter un radical néopentylène dans le cas où R est un radical $OR^4$, et $R^{11}$ représente un alkylène, un cycloalkylène ou un arylène, ou

X représente un radical éthylène ou propylène-1,3 ou un radical propylène-1,3 portant un méthyle en position 1 ou 2, et

R représente un radical répondant à l'une des formules suivantes:

$NR^6\overset{O}{\overset{\|}{C}}R^7$ (VIII), $R^8R^9N\overset{O}{\overset{\|}{C}}NR^{10}$ (IX)

et $(CH_3)_2N{-}X{-}NH\overset{O}{\overset{\|}{C}}NH{-}(Y{-}NH\overset{O}{\overset{\|}{C}}NH)_n$ (X)

dans lesquelles X et Y ont chacun, indépendamment l'un de l'autre, les significations qui leur ont été données ci-dessus, n est égal à 0 ou à 1, $R^6$ représente un atome d'hydrogène, un alkyle, un cycloalkyle, un cyano-2 éthyle ou un cyano-2 éthyle portant un méthyle en position 1 ou 2, $R^7$ représente un alkyle, un alcényle, un cycloalkyle, un aryle, un aralkyle, un alkylaryle ou un alkylarylalkyle, $R^8$ et $R^{10}$ représentent chacun un atome d'hydrogène et $R^9$ représente un alkyle, un cycloalkyle, un aryle, un aralkyle, un alkylaryle, un alkylarylalkyle ou un diméthylamino-3 propyle, ou encore $R^8$ et $R^9$ représentent chacun, indépendamment l'un de l'autre, un alkyle en $C_1$–$C_4$ ou un phényle et $R^{10}$ représente un atome d'hydrogène, ou $R^8$ représente un atome d'hydrogène, $R^9$ un alkyle, un cycloalkyle, un aryle, un aralkyle, un alkylaryle ou un arylalkylaryle et $R^{10}$ un cyano-2 éthyle ou un cyano-2 éthyle portant un méthyle en 1 ou 2.

2. Mélanges selon la revendication 1, caractérisés en ce que X représente un radical éthylène éventuellement porteur d'un alkyle en $C_1$–$C_{12}$ et R représente un radical répondant à l'une des formules II à VII.

3. Mélanges selon la revendication 1, caractérisés en ce que X, dans la formule I, représente un radical éthylène ou propylène-1,3 et R représente un radical répondant à l'une des formules VIII à X.

4. Mélanges selon la revendication 1, caractérisés en ce que $R^1$, dans la formule III, représente un alkyle en $C_1$–$C_{12}$, un cycloalkyle en $C_5$–$C_7$, ou un radical phényle ou benzyle substitué ou non.

5. Mélanges selon la revendication 1, caractérisés en ce que $R^2$ représente un atome d'hydrogène et $R^3$ un alkyle en $C_1$–$C_{12}$, un cycloalkyle en $C_5$–$C_7$, ou un radical phényle ou benzyle substitué ou non, ou $R^2$ et $R^3$ représentent chacun un alkyle en $C_1$–$C_4$, un cycloalkyle en $C_5$–$C_7$ ou un phényle.

6. Mélanges selon la revendication 1, caractérisés en ce que $R^4$ représente un radical alkyle ou

alcényle en $C_1$–$C_{18}$, de préférence en $C_1$–$C_{12}$, un cycloalkyle en $C_5$–$C_8$, un aryle en $C_6$–$C_{16}$, un aralkyle en $C_7$–$C_{16}$, un cyano-2 éthyle, un amino-3 propyle ou un diméthylamino-3 propyle.

7. Mélanges selon la revendication 1, caractérisés en ce que Y représente un alkylène en $C_1$–$C_{12}$, un cycloalkylène en $C_5$–$C_7$ ou un arylène en $C_6$–$C_{12}$.

8. Mélanges selon la revendication 1, caractérisés en ce que $R^6$ représente un alkyle en $C_1$–$C_4$ ou un cyclohexyle.

9. Mélanges selon la revendication 1, caractérisés en ce que $R^7$ représente un radical alkyle ou alcényle contenant de 1, à 18 atomes de carbone, de préférence de 1 à 12, un cycloalkyle en $C_5$–$C_7$, un aryle en $C_6$–$C_{12}$, un radical aralkyle ou alkylaryle en $C_7$–$C_{16}$, ou un alkylarylalkyle en $C_8$–$C_{16}$.

10. Mélanges selon la revendication 1, caractérisés en ce que $R^8$ et $R^{10}$ représentent chacun un atome d'hydrogène et $R^9$ représente un alkyle en $C_1$–$C_{12}$, un cycloalkyle en $C_5$–$C_7$, un aryle en $C_6$–$C_{12}$, un radical alkylaryle ou aralkyle en $C_7$–$C_{16}$ ou un alkylarylalkyle en $C_8$–$C_{16}$.

11. Mélanges selon la revendication 1, caractérisés en ce que $R^8$ représente un atome d'hydrogène, $R^9$ un alkyle en $C_1$–$C_{12}$, un cycloalkyle en $C_5$–$C_7$, un aryle en $C_6$–$C_{12}$, un radical alkylaryle ou aralkyle en $C_7$–$C_{16}$ ou un alkylarylalkyle en $C_8$–$C_{16}$, et $R^{10}$ représente un cyano-2 éthyle qui porte éventuellement un méthyle en position 1 ou en position 2.

12. Mélanges selon la revendication 1, caractérisés en ce que le composé diméthylaminé de formule I est:

$(CH_3)_2NCH_2CH_2OCH_2CH_2CN$,
$(CH_3)_2NCH_2CH_2OCOCH_3$,
$(CH_3)_2NCH_2CH_2CH_2NHCONHC_6H_5$,
$(CH_3)_2NCH_2CH_2OCON(C_2H_5)_2$
ou
$[(CH_3)_2NCH_2CH_2O]_2CO$